# EUROPEAN PATENT APPLICATION

(11) **EP 1 629 775 A1**
(43) Date of publication of application: **01.03.2006**
(21) Application number: 05719827.7
(22) Date of filing: 02.03.2005
(51) Int. Cl.: A61B 5/107

(54) **METHOD OF JUDGING DEGREE OF HAIR DAMAGING**

(30) Priority: 31.03.2004 JP 2004101687
(71) Applicant: Pola Chemical Industries Inc., Shizuoka-shi, Shizuoka 4200914 (JP)
(72) Inventor: MIYAMAE,Y. POLA CHEM.IND.INC. TOTSUKA-KENKYUSHO, Kanagawa 2440812 (JP); YAMAKAWA,Y. POLA CHEM.IND.INC. TOTSUKA-KENYUSHO, Kanagawa 2 440812 (JP)
(74) Representative: Bannerman, David Gardner
(86) International application number: PCT/JP2005/003512
(87) International publication number: WO 2005/096938

(57) **Abstract**

The present invention relates to a method of noninvasively and quantitatively evaluating a degree of a hair damage, specifically the degree of a hair damage caused by a permanent treatment and/or the degree of a damage caused by an oxidation treatment. The degree of a damage of a hair, whose degree of a damage is unknown, is evaluated on the basis of a correlation between the degree of a hair damage and a result of multivariate analysis near infrared absorption spectrum of the hair. The correlation can be obtained based on a result of multivariate analysis of near infrared absorption spectra of two or more kinds of hairs, whose degree of a damage is known. Furthermore, a hysteresis of treatment applied to the hair or the likelihood to be easily damaged by a treatment is determined from the obtained evaluation result. Principal component analysis (PCA), SIMCA, or KNN is preferably used as an algorithm of the multivariate analysis.

## Description

### TECHNICAL FIELD

The present invention relates to a method of evaluating the kind and degree of a hair damage. More specifically, the present invention relates to a method of evaluating the kind and degree of a hair damage from a result of multivariate analysis of a near infrared absorption spectrum of the hair.

### BACKGROUND ART

Hair damages are roughly classified into a morphological damage and a qualitative damage.
The morphological damage refers to a phenomenon in which the appearance and feeling of a hair deteriorate, such as peeling of cuticle, the occurrence of wrinkling on hair surface, or a flaw, trichorrhexis, or split hair. Examples of the morphological damage include a damage caused by friction, a damage caused by heat, and a damage caused by unskillful cutting.
On the other hand, the qualitative damage refers to a damage caused by a chemical change of a hair component. Examples of the qualitative damage include a damage caused by a permanent wave (a permanent treatment), a damage caused by a bleach or a hair color treatment, and a damage caused by light (such as ultraviolet light) exposure.

There are a large number of persons whose hairs are subjected toapermanentwave (permanent) treatment, or a bleaching treatment or a hair dye treatment. However, as described above, the hairs suffer qualitative damages owing to those treatments. If it becomes possible to exactly evaluate the degrees of the damages caused by those treatments, the damages can be appropriately recovered by selecting an appropriate hair cosmetic and the like.

In addition, a degree of likelihood to be easily damaged by the treatments varies depending on kinds of hairs (hairs of individuals). If the degree of likelihood can be predicted, the hair damages can be appropriately prevented.

Conventional methods of evaluating the degree of hair damage are classified into a noninvasive and an invasive method. Of those, the noninvasive method is preferable because a sample can be evaluated without being chemically or physically damaged.
Known as the invasive method is a method involving evaluation based on the tear strength of a hair collected from a subject (see JP-A 2002-282240), a diagnostic method involving the use of an immune reaction (see JP-A 6-265544), or the like. On the other hand, only a method involving visual sensory evaluation by a professional paneler has been known as the noninvasive method, or no noninvasive method of measuring the degree of hair damage qualitatively or quantitatively has been found.

Meanwhile, attempts have been made to determine the specific physical properties of a measuring object based on the results of spectral analysis obtained from a near infrared absorption spectrum of the measuring object. In those attempts, the physical properties are determined on the basis of a correlation between a specific characteristic value and a near infrared absorption spectrum, wherein the correlation is determined from the results of statistical processing of the near infrared absorption spectra of two or more kinds of samples whose specific characteristic values are known.
There has been reported the measurement of : the water content of a wood (see JP-A 11-509325); the kind and amount of an additive in a polymer (see JP-A 2004-53440); the water content of a skin (see JP-A 2002-90298); the water content of a hair (see JP-A 2003-344279) ; the presence or absence of mastitis (see the pamphlet of WO 01/075421); the smoothness and gloss of a hair (see JP-A 2003-270138); or the like by means of the method.

### DISCLOSURE OF THE INVENTION

### PROBLEMS TO BE SOLVED BY THE INVENTION

An object of the present invention is to provide a method of qualitatively and quantitatively evaluating the degree of a hair damage by using noninvasive means. More specifically, an object of the present invention is to provide a method of evaluating the degree of damage of a hair of an evaluating object, on the basis of a correlation between a result of multivariate analysis of near infrared absorption spectrum of a hair and the degree of damage of the hair. Here, the hair damage preferably includes a qualitative damage, or more preferably includes one of damages caused by a permanent treatment and by an oxidation treatment which is typified by a bleaching treatment or a hair dye treatment.

Another object of the present invention is to provide means for determining a hysteresis of treatment applied to a hair, for example, a hysteresis of permanent treatment or an oxidation treatment to the hair, by using the method of the present invention. Still another object of the present invention is to provide means for determining the hair as to its degree of likelihood to be easily damaged by the treatment.

### MEANS FOR SOLVING THE PROBLEMS

The inventors of the present invention have found that there is a correlation between a degree of a hair damage and a result of multivariate analysis of a near infrared absorption spectrum of the hair. They have also found that the degree of damage of a hair of an evaluating object can be evaluated from the near infrared absorption spectrum of the hair, on the basis of the correlation. That is, the present invention is as follows.
[1] A method of evaluating a degree of at least one of a damage of a hair caused by a permanent treatment and a damage of the hair caused by an oxidation treatment, based on a near infrared absorption spectrum of the hair, including the steps of:
   obtaining a correlation between the degree of at least one of the damage of a hair caused by the permanent treatment and the damage of the hair caused by the oxidation treatment, and a result of multivariate analysis of near infrared absorption spectrum (wavenumber region: 8,000 to 4,500 cm⁻¹) of the hair, based on results of the multivariate analysis of the near infrared absorption spectra of two or more kinds of hairs, a degree of at least one of damages caused by a permanent treatment and by an oxidation treatment is known;
   obtaining a near infrared absorption spectrum of a hair which is an evaluating object, a degree of at least one of a damages caused by the permanent treatment and by the oxidation treatment is unknown; and
   evaluating the degree of at least one of the damage caused by the permanent treatment and the damage caused by the oxidation treatment of the hair of the evaluating object, based on the near infrared absorption spectrum obtained in the step 2) and based on the obtained correlation.
[2] The method according to [1], wherein the multivariate analysis is analysis which uses a principal component analysis (PCA), SIMCA, or KNN.
[3] The method according to [1] or [2], wherein the damage caused by the oxidation treatment is a damage caused by a bleaching treatment.
[4] The method according to [1], wherein the method is for determining a degree of the permanent treatment and/or the oxidation treatment applied to the hair.
[5] The method according to [1], wherein the method is for determining the hair as to its degree of likelihood to be easily damaged by the permanent treatment and/or the oxidation treatment.

### EFFECT OF THE INVENTION

According to the method of the present invention, the degree of hair damage can be evaluated qualitatively and quantitatively, and noninvasively. The method can be used for determining a hysteresis of treatment applied to a hair, or for determining a hair as to its degree of likelihood to be easily damaged by the treatment.
Therefore, the method of the present invention can be used to repair and prevent hair damage.

### BRIEF DESCRIPTION OF THE DRAWINGS

Fig. 1 is a two-dimensional scatter diagram created from results of multivariate analysis in Example 1 (wavenumber region of a spectrum: 5, 060 to 4, 500 cm⁻¹; data processing: mean centering, SNV, and secondary differentiation).
Fig. 2 is a two-dimensional scatter diagram created from results of multivariate analysis in Example 2 (wavenumber region of a spectrum: 5, 060 to 4, 500 cm⁻¹; data processing: mean centering, SNV, and secondary differentiation).
Fig. 3 is a two-dimensional scatter diagram created from results of multivariate analysis in Example 3 (wavenumber regions of a spectrum: 6,000 to 5,500 cm⁻¹ and 5,060 to 4,500 cm⁻¹; data processing: mean centering, SNV, and secondary differentiation).
Fig. 4 is a two-dimensional scatter diagram created from results of multivariate analysis in Example 4 (wavenumber region of a spectrum: 6, 000 to 5, 500 cm⁻¹; data processing: mean centering, SNV, and secondary differentiation).
Fig. 5 is a two-dimensional scatter diagram created from results of multivariate analysis in Example 5 (wavenumber region of a spectrum: 8, 000 to 6, 000 cm⁻¹; data processing: mean centering, SNV, and secondary differentiation).
Fig. 6 is a two-dimensional scatter diagram created from results of multivariate analysis in Comparative Example 1 (wavenumber region of a spectrum: 8,000 to 4,000 cm⁻¹; data processing: mean centering, SNV, secondary differentiation).
Fig. 7 is a two-dimensional scatter diagram created from results of multivariate analysis in Comparative Example 2 (wavenumber region of a spectrum: 4,500 to 4,000 cm⁻¹; data processing: mean centering, SNV, and secondary differentiation).
Fig. 8 is a two-dimensional scatter diagram created from results of multivariate analysis in Comparative Example 3 (wavenumber region of a spectrum: 5,060 to 4,500 cm⁻¹; data processing: mean centering, SNV, and secondary differentiation).
Fig. 9 is a two-dimensional scatter diagram created from results of multivariate analysis in Comparative Example 4 (wavenumber region of a spectrum: 5,060 to 4,500 cm⁻¹; data processing: mean centering, MSC, and secondary differentiation) .
Fig. 10 is a two-dimensional scatter diagram created from results of multivariate analysis in Comparative Example 5 (wavenumber region of a spectrum: 5,060 to 4,500 cm⁻¹; data processing: mean centering, SNV, and primary differentiation).
Fig. 11 is a diagram showing a relationship between a degree of damage of each of multiple untreated hair samples, and a degree of damage of each of samples obtained by applying a permanent treatment and/or a bleaching treatment to the multiple untreated hair samples.

### BEST MODE FOR CARRYING OUT THE INVENTION

As described above, the method of the present invention is a method of evaluating a degree of at least one of a damage of a hair caused by a permanent treatment and a damage of the hair caused by an oxidation treatment.

The permanent treatment is also referred to as a permanent wave. The permanent treatment is generally performed by using a permanent agent 1 containing a reducing agent and a permanent agent 2 containing an oxidant. Examples of the reducing agent in the permanent agent 1 include a thioglycol-based agent, a thiolactic acid-based agent, and a cysteine-based agent (such as acetylcysteine). The permanent agent 1 preferably contains an alkali agent in addition to the reducing agent. Examples of the oxidant in the permanent agent 2 include hydrogen peroxide and bromate.

The permanent treatment can involve: treating a hair with the permanent agent 1 (preferably applying the agent 1 to the hair) to cleave at least part of an S-S bond (disulfide bond) of keratin in the hair; and treating the hair treated with the permanent agent 1, with the permanent agent 2 (preferably applying the agent 2 to the hair) to recombine the cleaved S-S bond.
The permanent treatment allows the hair to be changed semipermanently, but imparts some degree of damage to the hair. A specific example of the damage, which can be a damage due to the denaturation of a protein in the hair, may include a hydrolysis of an amide bond (ex. -COONH- → -COOH + NH₂) and a failure of recombination of an S-S bond (disulfide bond) cleaved by a reducing action (ex. -SS- → -SH).

On the other hand, the phrase "damage of a hair caused by an oxidation treatment" means each of damages that a hair treated with a treatment agent containing an oxidant suffers and that a hair irradiated with ultraviolet light suffers. That is, examples of the oxidation treatment include a bleaching treatment, a hair dye treatment, and a treatment involving irradiation with ultraviolet light. The examples of the oxidation treatment further include bringing a hair into contact with water containing chlorine or perchloric acid (such as water of a swimming pool).
The damage caused by an oxidation treatment is preferably a damage caused by a bleaching treatment or a hair dye treatment.

The bleaching treatment is also referred to as a decoloring treatment. The bleaching treatment can be performed by applying a bleaching agent to a hair. An oxidant in the bleaching agent is preferably hydrogen peroxide. A perhydroxy anion generated from hydrogen peroxide can decompose a melanin pigment. The bleaching agent may contain an alkali agent (such as ammonia or monoethanolamine) or a pro-oxidant in addition to the oxidant. The bleaching treatment may be performed as a pretreatment of a hair dye treatment.
When a hair is treated with a bleaching agent (preferably the agent is applied to the hair), a melanin pigment in the hair can be decomposed to bleach the hair, and meanwhile the hair is damaged. The damage is considered to be a damage mainly due to the denaturation of a protein. A specific example of the damage may include the cleavage of an S-S bond (disulfide bond) by an oxidant (ex. R-S-S-R → R-SO-S-R → R-SO₂-S-R → [R-SO₂-SO-R] → R-SO₂-SO₂-R- → 2R-SO₃H) . Another specific example of the damage may include a strain occurring in a cuticula pili by virtue of repetition of the swelling and softening of a hair, in addition to an oxidative decomposition of a melanin pigment (i.e. decoloring) by hydrogen peroxide.

The hair dye treatment is also referred to as a treatment for coloring hair. The hair dye treatment can be performed by applying a hair dye agent to a hair. The hair dye agents are classified into a permanent hair dye, a semipermanent hair dye, and a temporary hair dye. Of those, a permanent hair dye is preferable in the present invention.
The hair dye (preferably a permanent hair dye) agent preferably contains an oxidant, an alkali agent or the like in addition to a dye. The oxidant in the hair dye agent is preferably hydrogen peroxide, and the alkali agent is preferably ammonia or the like.
A hair is dyed with a hair dye agent (preferably the agent is applied to the hair). Meanwhile, the hair is damaged to some degree with the hair dye agent. The damage is considered to be a damage mainly due to the denaturation of a protein in a hair. Specific examples of the damage may include the cleavage of an S-S bond (disulfide bond) by an oxidant (R-S-S-R → R-SO-S-R → R-SO₂-S-R → [R-SO₂-SO-R] → R-SO₂-SO₂-R- → 2R-SO₃H) and the forming of an azine bond through an oxidation reaction between an oxidation dye and a protein in a hair. The examples of the damage may further include a strain occurring in a cuticula pili by virtue of repetition of the swelling and softening of a hair, in addition to an oxidative decomposition of a melanin pigment (i.e. decoloring) by hydrogen peroxide.

In addition, the oxidation treatment involves ultraviolet light irradiation of a hair, for example, exposure of the hair to sunlight. A damage mainly due to the denaturation of a protein (keratin or the like) occurs in the hair irradiated with ultraviolet light.

The method of the present invention is a method of evaluating the degree of hair damage from a near infrared absorption spectrum of the hair. The degree of the hair damage to be evaluated includes at least one of the degree of the damage caused by a permanent treatment and the degree of the damage caused by an oxidation treatment described above.

The evaluation method of the present invention includes the steps of:
1) obtaining a correlation between a degree of at least one of a damage of a hair caused by a permanent treatment and a damage of the hair caused by an oxidation treatment, and a result of multivariate analysis of near infrared absorption spectrum of the hair, based on results of the multivariate analysis of the near infrared absorption spectra of two or more kinds of hairs, a degree of at least one of a damages caused by a permanent treatment and by an oxidation treatment is known;
2) obtaining a near infrared absorption spectrum of a hair which is an evaluating object, a degree of at least one of a damages caused by a permanent treatment and by an oxidation treatment is unknown; and
3) evaluating the degree of at least one of the damage caused by the permanent treatment and the damage caused by the oxidation treatment of the hair of the evaluating object, based on the near infrared absorption spectrum obtained in the step 2) and based on the obtained correlation.

The near infrared absorption spectrum of a hair in the step 1) can be obtained by means of an arbitrary means. The near infrared absorption spectrum of the hair can be measured with various types of near infrared absorption spectrum measuring devices.
For example, the measurement can be performed with a dispersive measuring device employing a diffraction grating or a measuring device employing a diode array as a detector. In addition, the measured near infrared absorption spectrum of the hair may be subjected to Fourier transformation.

The near infrared absorption spectrum of the hair obtained in the step 1) is subjected to multivariate analysis. The term "multivariate analysis" intends pattern recognition for analyzing a relationship of each sample among samples (hair samples in the present invention) on the basis of multiple observations (here, near infrared absorption spectral values) through calculating a similarity and the like.

The multivariate analysis is preferably performed in accordance with the following steps.
a) Near infrared absorption spectra of two or more kinds of hairs are subjected to data-processing as required.
b) A matrix is created, in which spectral values of the near infrared absorption spectra or the data-processing spectra (hereinafter, they are collectively referred to as "spectra") for every divided wavenumber region are arranged in a column, and the degree of a damage of a hair caused by a permanent treatment and the degree of a damage of the hair caused by an oxidation treatment are arranged in a row.
c) The created matrix is subjected to multivariate analysis to properly derive the first and second components.
d) A relationship of each sample is obtained by using the first component as a first axis and the second component as a second axis.

A wavenumber region of the near infrared absorption spectrum or the near infrared absorption spectrum to be subjected to data processing in the step a) is preferably a region consisting of at least part of 8,000 to 9, 500 cm⁻¹, or more preferably a region consisting of at least part of 6,000 to 4,500 cm⁻¹.
A near infrared absorption spectrum of a hair in the wavenumber region is considered to exactly grasp the state of existence and behavior of a protein in the hair. Therefore, the degrees of a hair damage caused by a permanent treatment and a hair damage caused by an oxidation treatment are probably exactly reflected in the spectrum in the wavenumber region. Examples described below also show that there is a clear correlation between a result of multivariate analysis of the near infrared absorption spectrum or the near infrared absorption spectrum subjected to data processing in the wavenumber region, and the degree of the hair damage.

The data processing in the step a) includes pretreatment and transformation.
Examples of the pretreatment include autoscaling, mean centering, range scaling, and variance scaling.
Examples of the transformation include primary differentiation, high-order differentiation (including secondary differentiation), standard normal variant (SNV), multiplicative scatter correction (MSC), normalization, smoothing, subtraction, common logarithm (log10), multiplication, and baseline correction.

The data processing in the step a) preferably includes secondary differentiation, more preferably includes standard normal variant (SNV) and secondary differentiation, or still more preferably includes mean centering, standard normal variant (SNV), and secondary differentiation.
Such processing enables variations of individual differences to be corrected, and influences of a noise, an outlier and the like to be removed. As a result, quality of data from the spectra can be enhanced.

In any case, the data processing in the step a) is preferably performed in such a manner that the first and second components, derived in the step c) from the matrix created in the step b) to be described later, have clearer correlation with the degrees of a damage of the hair caused by a permanent treatment and a damage of the hair caused by an oxidation treatment.

A column in the matrix created in the step b) shows spectral values for every divided wavenumber region of the spectrum of respective hairs. The spectral value refers to: an absorbance for a near infrared absorption spectrum not subjected to a transformation treatment; or a differentiated value of an absorbance for a differentiated spectrum.
Here, the division of a spectrum is preferably performed at constant interval of wavenumber. The interval of wavenumber is not particularly limited. The spectrum is preferably divided every wavenumber of generally 2 to 16 cm⁻¹, preferably 4 to 8 cm⁻¹ (4 or 8 cm⁻¹ when a resolution is 4 cm⁻¹), or more preferably 4 cm⁻¹. A spectral value for every divided wavenumber region of a spectrum is desirably represented by an average of spectral value for the divided wavenumber region.

A row in the matrix created in the step b) shows the degree of damage of each of two or more kinds of hairs, the spectrum of which has been measured (the degree of a damage caused by a permanent treatment and/or a damage caused by an oxidation treatment). Here, the degree of a hair damage may be indicated by a degree of a treatment applied to the hair. The degree of a treatment applied to a hair means: the number of treatments; the concentration of an active ingredient in a treatment agent used for the treatment; a treating time; or the like.
Thus, a matrix is created by obtaining spectral values for every divided wavenumber region of the respective spectra obtained from two or more kinds of hairs.

Principal component analysis (PCA), SIMCA, or KNN is preferably used as an algorithm of the multivariate analysis in the step c). It is needless to say that the first component and the second component derived from the matrix by the multivariate analysis are independent of each other, that is, the respective vectors are orthogonal to each other.
The first component and the second component derived have correlations with the degree of a damage of a hair caused by a permanent treatment and the degree of a damage of the hair caused by an oxidation treatment.

Furthermore, if the third component is derived as required, the degree of a hair damage other than the hair damages caused by a permanent treatment and by an oxidation treatment may be evaluated. Examples of the hair damage other than the damages caused by a permanent treatment and an oxidation treatment include morphological damages (including a damage caused by friction, heat, or unskillful cutting).

The step d) is a step of obtaining a relationship of each sample by using at least two components derived in the step c) as axes. For example, a two-dimensional scatter diagram having two components as axes is created, and the relationship of each sample can be obtained from a positional relationship of a plot corresponding to each sample. A correlation between the degree of a hair damage and a result of multivariate analysis of a spectrum of the hair can be obtained from the relationship of each sample.
Grouping with reference to the degree of hair damage may be performed on the basis of the resultant relationship of the each sample. The grouping can be performed by using an algorithm of SIMCA or the like.

The result of multivariate analysis obtained in the step 1) has a correlation with a damage of a hair caused by a permanent treatment and a damage of the hair caused by an oxidation treatment. That is, in the obtained result of multivariate analysis, one of the at least two components derived (referred to as a component A) shows a correlation with the degree of the damage of the hair caused by the permanent treatment, and the other component (referred to as a component B) shows a correlation with the degree of the damage of the hair caused by the oxidation treatment.
The axis of the component A shows the degree of a damage caused by a permanent treatment, therefore a correlation between the degree of the damage caused by the permanent treatment and the result of the multivariate analysis of the spectrum can be obtained through observing a relationship between an intact hair (that is, a hair not subjected to a permanent treatment) and a hair subjected to a permanent treatment with respect to the axis of the component A.
Similarly, the axis of the component B shows the degree of a damage caused by an oxidation treatment, therefore a correlation between the degree of the damage caused by the oxidation treatment and the result of the multivariate analysis of the spectrum can be obtained through observing a relationship between an intact hair (that is, a hair not subj ected to an oxidation treatment) and a hair subjected to a permanent treatment with respect to the axis of the component B.

In addition, from loading plots for various wavenumber regions of spectra, the degree to which a change in spectral value in each wavenumber region of spectra is involved in a change indicated by a main component axis is turned out. Therefore, the degree of the hair damage can be interpreted as a specific chemical change, that is, peak change.

As described above, the method of the present invention includes the step of 2) obtaining a near infrared absorption spectrum of a hair which is an evaluating object, a degree of at least one of damages caused by a permanent treatment and by an oxidation treatment is unknown. The near infrared absorption spectrum in the step 2) is preferably obtained with the same method or device as that used in the measurement of the near infrared absorption spectrum in the step 1). Furthermore, the resultant near infrared absorption spectrum is preferably subjected to data processing as in 1).

As described above, the present invention includes the step of 3) evaluating the degree of at least one of the damage caused by the permanent treatment and the damage caused by the oxidation treatment of the hair of the evaluating object, based on the near infrared absorption spectrum obtained in the step 2) and based on the obtained correlation.
That is, the spectral matrix data, the correlation from which has been obtained in 1), and the spectrum data obtained in 2), are collectively subjected to multivariate analysis in the same manner as in 1), whereby the degree of damage of the hair of the evaluating object in 2) is evaluated (principal component analysis). Alternatively, the spectrum data obtained in 2) is applied to a model obtained from the correlation obtained in 1), whereby the degree of damage of the hair as the evaluating object in 2) is evaluated (SIMCA or KNN).
The degrees of a damage caused by a permanent treatment and a damage caused by an oxidation treatment of the hair whose damaged state is unknown, can be evaluated by confirming a relationship between a result of analysis of a hair whose damaged state is known and a result of analysis of the hair whose damaged state is unknown, with respect to the axes of the components A and B. The relationship with respect to the axes of the components A and B can be represented as, for example, a two-dimensional scatter diagram having the components A and B as axes.

The method of evaluating the degree of a hair damage of the present invention can be used for determining a degree of a permanent treatment and/or an oxidation treatment applied to the hair. The degree of a permanent treatment and/or an oxidation treatment applied to the hair means hysteresis of the permanent treatment or the oxidation treatment applied to the hair and the contents of the treatment. The contents of the treatment mean: the concentration of an active ingredient in a treatment agent used for the treatment; the number of treatments; a treating time; and the like.
That is, the degrees of those treatments can be determined from evaluation results obtained by the method of the present invention.

In addition, the method of evaluating the degree of a hair damage of the present invention can be used for determining the hair as to its degree of likelihood to be easily damaged by a permanent treatment and/or an oxidation treatment. The degree of likelihood to be easily damaged means the degree of a damage that a hair will suffer when the hair is subjected to a permanent treatment and/or an oxidation treatment.
A relative relationship of a determining object hair, which has been evaluated to have a low degree of damage (for example, judged to be untreated) by the method of the present invention, within a group of multiple untreated hair samples is confirmed, whereby the object hair as to its degree of likelihood to be easily damaged can be determined. In the case where a hair, which has been evaluated to be untreated, is evaluated to have a relatively high degree of damage within a group of untreated hair samples, it can be determined that the hair is susceptible to the treatment and easily damaged. On the other hand, in the case where the hair is evaluated to have a relatively low degree of damage within the group of untreated hair samples, it can be determined that the hair is not susceptible to the treatment. This will also be shown in Examples described below.

Hereinafter, the present invention will be described in detail with reference to examples and the like, but the scope of the present invention is not limited to these examples.

### <Preparation of hair sample>

11 subjects who had volunteered were randomly divided into a permanent treatment group (6 persons) and a bleaching treatment group (5 persons) . 3 bundles of hairs (diameter: 7 to 8 mm) were collected from each of the subjects (11 persons). The collected bundles were treated with a permanent agent and/or a bleaching agent as shown below.
The permanent agent and bleaching agent used are specifically as follows.
5% permanent agent: an aqueous solution containing 5 mass % of ammonium thioglycolate and an aqueous solution containing 7 mass% of sodium bromate
10% permanent agent: an aqueous solution containing 10 mass% of ammonium thioglycolate and an aqueous solution containing 7 mass% of sodium bromate
Bleaching agent: an aqueous solution containing 3 mass% of hydrogen peroxide and 3 mass% of ammonia

### PREPARATION 1 OF HAIR SAMPLE

One of the 3 bundles of hairs collected from each of the subjects (6 persons) in the permanent treatment group was treated with the 5% permanent agent. Another one was treated with the 10% permanent agent. The remaining one remained untreated.
Treatment with a permanent agent was performed in accordance with the following procedure.
1) About 10 bundles of hairs (each having a diameter of about 7 mm) were placed in a 500-ml beaker. Furthermore, an aqueous solution of ammonium thioglycolate was charged so that the hairs were immersed in it. The immersed hairs were left standing for 5 min at about 29°C. The resultant bundles of hairs were washed with running water for about 3 min.
2) The bundles of hairs obtained in 1) were placed in a 500-ml beaker. Furthermore, an aqueous solution of sodium bromate was charged so that the hairs were immersed in it. The immersed hairs were left standing for 10 min, and were then washed with running water for about 3 min.
3) The bundles of hairs obtained in 2) and the untreated bundle of hairs were dried by means of a drier at 40°C. The resultant hair samples were referred to as "5% permanent treated sample", "10% permanent treated sample", and "untreated sample", respectively.

### PREPARATION 2 OF HAIR SAMPLE

Each of the 5% permanent treated samples and the 10% permanent treated samples obtained in PREPARATION 1 was additionally treated with the bleaching agent once. To be specific, about 10 bundles of hairs were placed in a 500-ml beaker. Furthermore, the bleaching agent was charged into the beaker, and the hairs were immersed in the agent. The immersed hairs were left standing for 20 min, and were then washed with running water for about 3 min. The washed bundles of hairs were dried by means of a drier at 40°C.
The resultant hair samples were referred to as "5% permanent treated + bleaching treated sample" and "10% permanent treated + bleaching treated sample", respectively.

### PREPARATION 3 OF HAIR SAMPLE

One of the 3 bundles of hairs collected from each of the subjects (5 persons) in the bleaching treatment group was treated with the bleaching agent once. Another one was treated with the bleaching agent 3 times. The remaining one remained untreated.
To be specific, about 10 bundles of hairs were placed in a 500-ml beaker. Furthermore, the bleaching agent was charged into the beaker, and the hairs were immersed in the agent. The immersed hairs were left standing for 20 min, and were then washed with running water for about 3 min. This procedure was repeated once or 3 times.
The resultant treated bundles of hairs and the untreated bundle of hairs were dried by means of a drier at 40°C.
The resultant hair samples were referred to as "once bleaching treated sample" and "3 times bleaching treated sample", respectively.

### <Measurement of near infrared absorption spectrum of hair sample>

The near infrared absorption spectra of the hair samples prepared in PREPARATIONS 1 to 3 OF HAIR SAMPLES were measured in a constant environment of 20°C. Here, near infrared absorption spectra of randomly selected 6 to 10 spots of each of hair sample were measured, in consideration of the possibility that the treatment varied from spot to spot of the hair sample.
A Fourier transform near infrared spectrophotometer VECTOR 22/N (manufactured by Bruker Optics Inc.) was used for measuring a near infrared absorption spectrum of the hair samples. The measurement conditions included: a resolution of 8 cm⁻¹; and a measurement wavenumber of 8,000 to 4,000 cm⁻¹. A diffuse reflection method using a fiber probe was employed.

### <Example 1>

The near infrared absorption spectra of the hair samples prepared in PREPARATIONS 1 to 3 OF HAIR SAMPLE were subjected to data processing for a wavenumber region 5,060 to 4,500 cm⁻¹. To be specific, after subjecting the spectrum to mean centering, standard normal variant and secondary differentiation were performed.

The spectra subjected to data processing were divided every 4 cm⁻¹, and the spectral value for each divided spectrum (secondary differentiated value of an absorbance) was calculated. A matrix was created, in which the calculated spectral values were arranged in rows and the contents of the treatment applied to the hair (no treatment, 5% or 10% permanent treatment, bleaching treatment once or 3 times, or combination of a permanent treatment and a bleaching treatment) were arranged in columns. The created matrix was subjected to multivariate analysis using principal component analysis. A two-dimensional scatter diagram having a first principal component as an axis 1 and a second principal component as an axis 2 was created from the obtained results of the analysis.
The data processing and the principal component analysis were performed by using multivariate analysis software (PIROUETTE version 3.11; manufactured by GL Sciences Inc.).

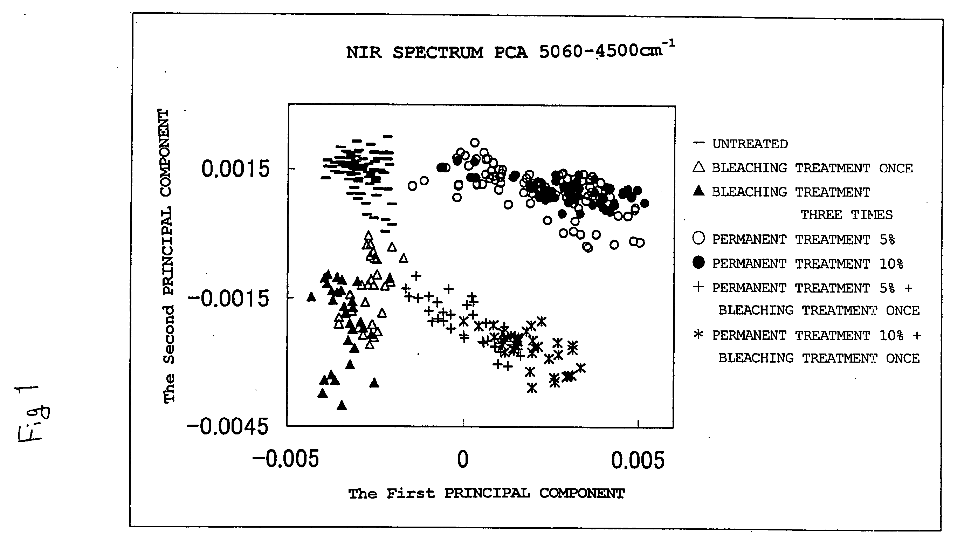

Fig. 1 shows the two-dimensional scatter diagram created from the obtained results of the multivariate analysis. As shown in Fig. 1, untreated, bleaching, permanent, and composite (permanent + bleaching) treated groups were classified very clearly.
To be specific, it can be understood that the degree of a damage caused by a permanent treatment increases as a value of the axis of the first principal component (Factor 1) increases. Furthermore, it can be understood that the value of Factor 1 becomes higher for a hair subjected to a permanent treatment with a permanent agent having a higher concentration of ammonium thioglycolate.
It can also be understood that the degree of a damage due to a bleaching treatment increases as a value of the axis of the second principal component (Factor 2) decreases. Furthermore, it can be understood that the value of Factor 2 decreases as the number of bleaching treatments to which a hair is subjected increases.
Therefore, it can be understood that the degrees of a damage of a hair due to a permanent treatment and a damage of the hair due to an oxidation treatment (bleaching treatment) show clear correlations with resultsof multivariate analysis of NIR spectra.

### <Example 2>

The near infrared absorption spectra of the untreated samples, the three times bleaching treated samples, the 10% permanent treated samples, and the 10% permanent treated + bleaching treated samples out of the hair samples, and a near infrared absorption spectrum of a bundle of hairs whose degree of damages caused by a permanent treatment and by an oxidation treatment are unknown, were subjected to data processing and principal component analysis in the same manner as in Example 1.

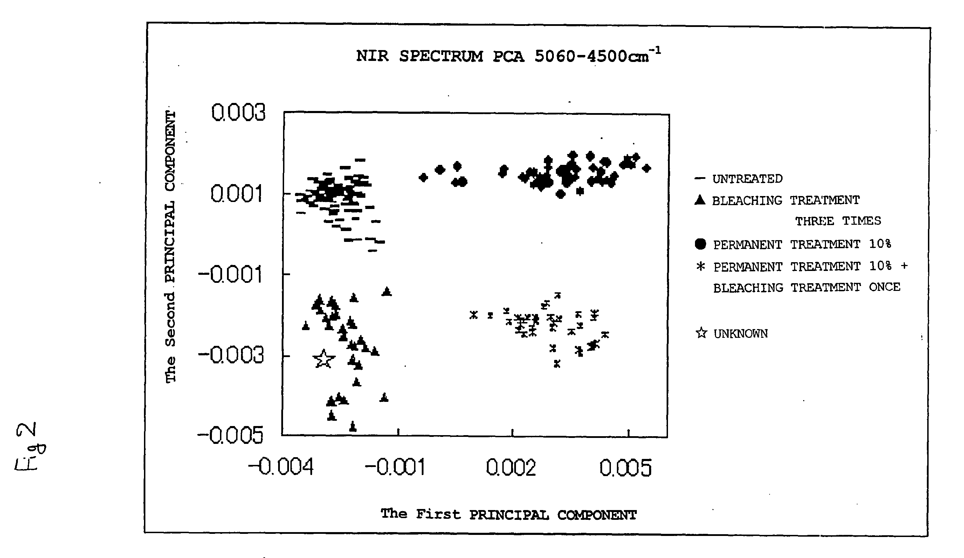

Fig. 2 shows the scatter diagram created from the obtained results of the analysis. As shown in Fig. 2, the respective sample groups having different contents of treatments were classified very clearly. In addition, the plot position of the result of the bundle of hairs whose degree of damages was unknown allowed one to evaluate that the hairs were subjected to bleaching treatment 3 times.

### <Example 3>

The near infrared absorption spectra of the untreated samples, the 3 times bleaching treated samples, the 10% permanent treated samples, and the 10% permanent treated + bleaching treated samples out of the hair samples were subjected to data processing and principal component analysis in the same manner as in Example 1, except that the wavenumber region of a spectrum to be analyzed was changed from 5, 060 to 4,500 cm⁻¹ to 6, 000 to 5, 500 and 5, 060 to 4, 500 cm-¹.

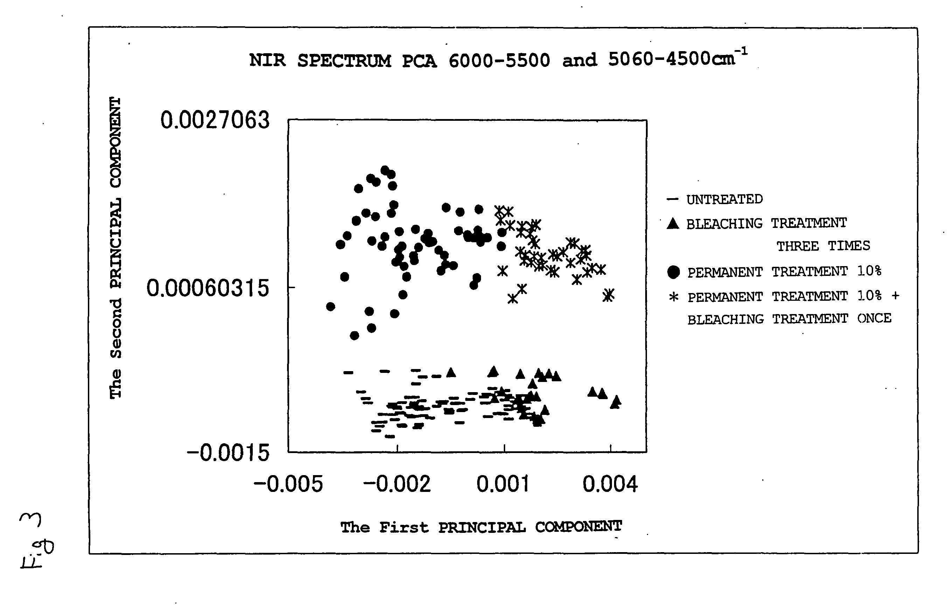

Fig. 3 shows the scatter diagram created from the obtained results of the analysis. As shown in Fig. 3, the respective sample groups having different contents of treatments were classified clearly.
Therefore, the near infrared absorption spectrum of a hair sample whose degree of damage is unknown is similarly subjected to data processing and multivariate analysis together with the above samples, whereby the degree of a damage of the hair sample whose degree of damage is unknown can be evaluated.

### <Example 4>

The near infrared absorption spectra of the untreated samples, the three times bleaching treated samples, the 10% permanent treated samples, and the 10% permanent treated + bleaching treated samples out of the hair samples were subjected to data processing and principal component analysis in the same manner as in Example 1, except that the wavenumber region of a spectrum to be analyzed was changed from 5,060 to 4,500 cm⁻¹ to 6,000 to 5,500 cm⁻¹.

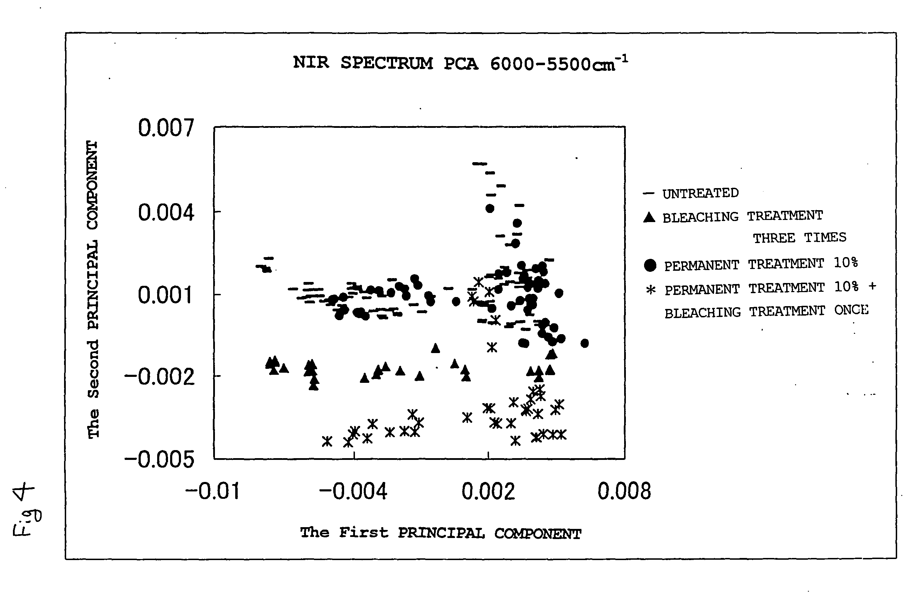

Fig. 4 shows the scatter diagram created from the obtained results of the analysis. As shown in Fig. 4, the respective sample groups having different contents of treatments were classified clearly.
Therefore, the near infrared absorption spectrum of a hair sample whose degree of damage is unknown is similarly subjected to data processing and multivariate analysis together with the above samples, whereby the degree of a damage of the hair sample whose degree of damage is unknown can be evaluated.
It can also be understood that the classification is less clear than the classification in the case where a spectrum in a wavenumber region of 5,060 to 4,500 cm⁻¹ is analyzed.

### <Example 5>

The near infrared absorption spectra of the untreated sample, the 3 times bleaching treated sample, the 10% permanent treated sample, and the 10% permanent treated + bleaching treated sample out of the hair samples were subjected to data processing and principal component analysis in the same manner as in Example 1, except that the wavenumber region of a spectrum to be analyzed was changed from 5,060 to 4,500 cm⁻¹ to 8,000 to 6,000 cm⁻¹.

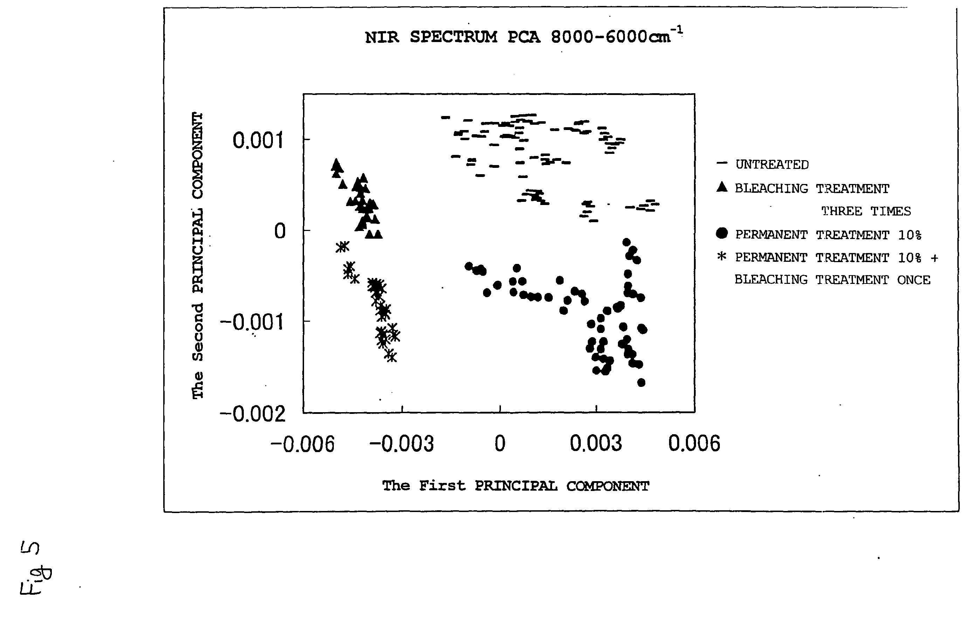

Fig. 5 shows the scatter diagram created from the obtained results of the analysis. As shown in Fig. 5, the respective sample groups having different contents of treatments were classified clearly.
Therefore, the near infrared absorption spectrum of a hair sample whose degree of damage is unknown is similarly subjected to data processing and multivariate analysis together with the above samples, whereby the degree of a damage of the hair sample whose degree of damage is unknown can be evaluated.
It can also be understood that the classification is less clear than the classification in the case where a spectrum in a wavenumber region of 5,060 to 4,500 cm⁻¹ is analyzed.

### <Comparative Example 1>

The near infrared absorption spectra of the untreated sample, the three times bleaching treated sample, the 10% permanent treated sample, and the 10% permanent treated + bleaching treated sample out of the hair samples were subjected to data processing and principal component analysis in the same manner as in Example 1, except that the wavenumber region of a spectrum to be analyzed was changed from 5,060 to 4,500 cm⁻¹ to 8,000 to 4,000 cm⁻¹.

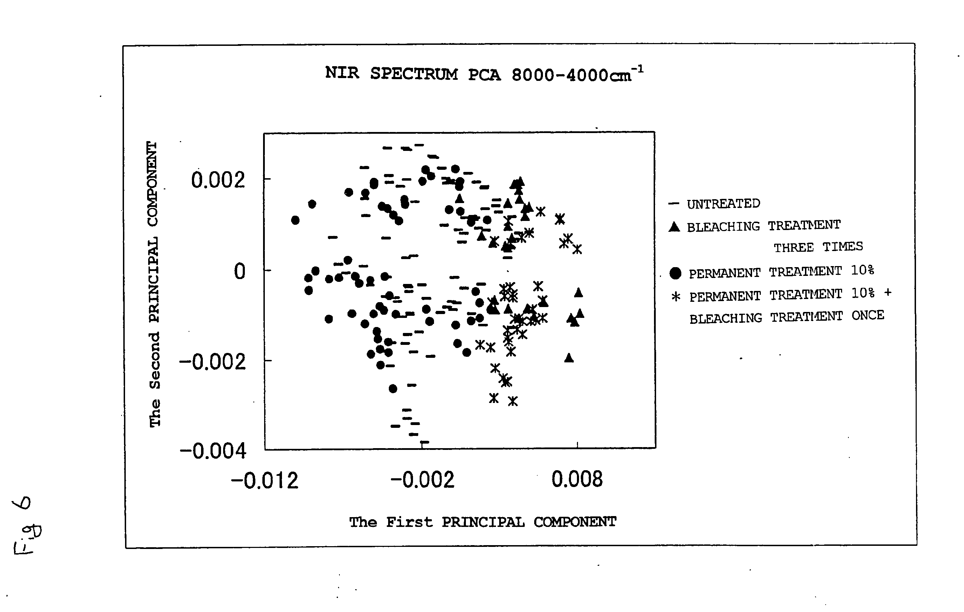

Fig. 6 shows the scatter diagram created from the obtained results of the analysis. As shown in Fig. 6, the respective sample groups having different contents of treatments were not sufficiently classified.

### <Comparative Example 2>

The near infrared absorption spectra of the untreated sample, the 3 times bleaching treated sample, the 10% permanent treated sample, and the 10% permanent treated + bleaching treated sample out of the hair samples were subjected to data processing and principal component analysis in the same manner as in Example 1, except that the wavenumber region of a spectrum to be analyzed was changed from 5,060 to 4,500 cm⁻¹ to 4,500 to 4,000 cm⁻¹.

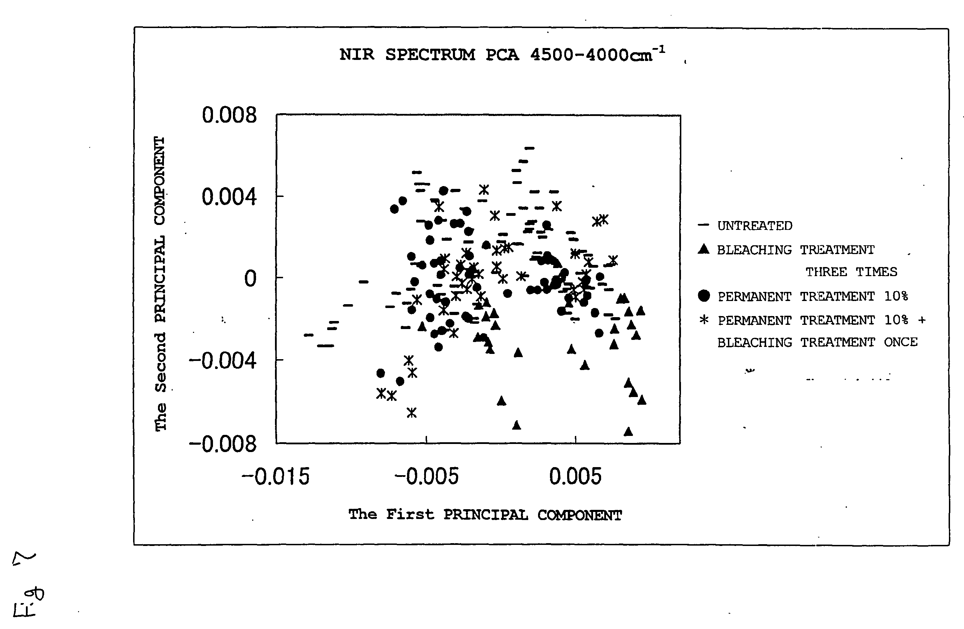

Fig. 7 shows the scatter diagram created from the obtained results of the analysis. As shown in Fig. 7, the respective sample groups having different contents of treatments were not sufficiently classified.

Table 1 summarizes the results of Examples 1 to 5 and Comparative Examples 1 and 2.

**[Table 1]**

| | Wavenumber region used | Result of classification |
|---|---|---|
| Example 1 | 5060-4500 | ○ |
| Example 2 | 5060-4500 | ○ |
| Example 3 | 6000-5500 and 5060-4500 | ○ |
| Example 4 | 6000-5500 | Δ |
| Example 5 | 8000-6000 | Δ |
| Comparative Example 1 | 8000-4000 | × |
| Comparative Example 2 | 4500-4000 | × |

### <Comparative Example 3>

The near infrared absorption spectra of the untreated samples, the three times bleaching treated samples, the 10% permanent treated samples, and the 10% permanent treated + bleaching treated samples out of the hair samples were subjected to data processing for a wavenumber region of 5,060 to 4,500 cm⁻¹. To be specific, after subjecting the spectrum to mean centering, secondary differentiation was performed. The spectra subjected to data processing were subjected to principal component analysis in the same manner as in Example 1.

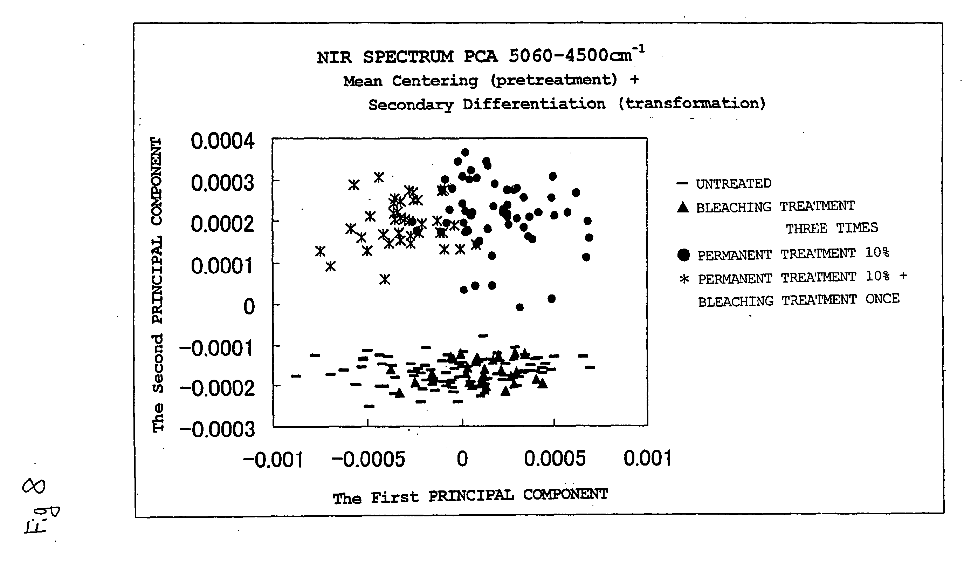

Fig. 8 shows the scatter diagram created from the obtained results of the analysis. As shown in Fig. 8, the respective sample groups having different contents of treatments were not sufficiently classified.

### <Comparative Example 4>

The near infrared absorption spectra of the untreated samples, the three times bleaching treated samples, the 10% permanent treated samples, and the 10% permanent treated + bleaching treated samples out of the hair samples were subjected to data processing for a wavenumber region of 5,060 to 4,500 cm⁻¹. To be specific, after subjecting the spectrum to mean centering, multiplicative scatter correction (MSC) and secondary differentiation were performed. The spectra subjected to data processing were subjected to principal component analysis in the same manner as in Example 1.

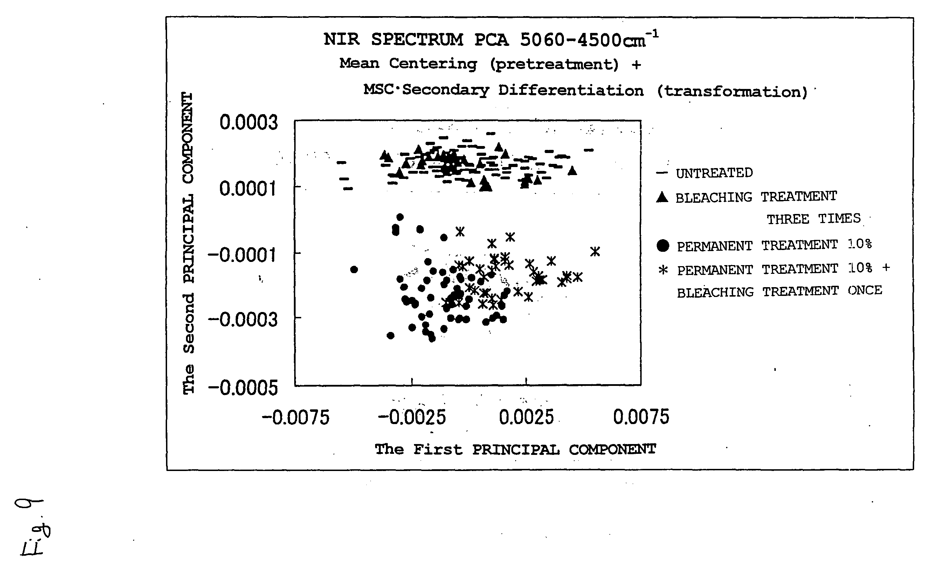

Fig. 9 shows the scatter diagram created from the obtained results of the analysis. As shown in Fig. 9, the respective sample groups having different contents of treatments were not sufficiently classified.

### <Comparative Example 5>

The near infrared absorption spectra of the untreated samples, the three times bleaching treated samples, the 10% permanent treated samples, and the 10% permanent treated + bleaching treated samples out of the hair samples were subjected to data processing for a wavenumber region of 5,060 to 4,500 cm⁻¹. To be specific, after subjecting the spectrum to mean centering, standard normal variate (SNV) and primary differentiation were performed. The spectra subjected to data processing were subjected to principal component analysis in the same manner as in Example 1.

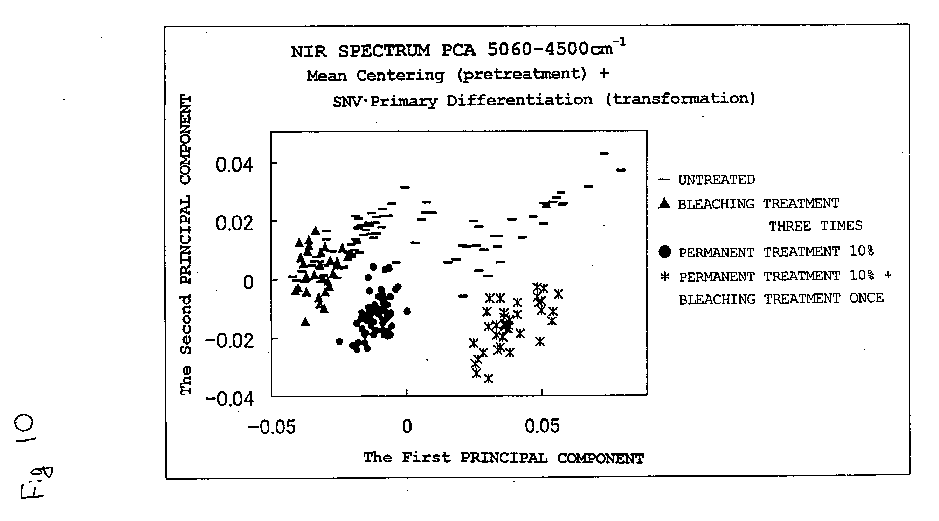

Fig. 10 shows the scatter diagram created from the obtained results of the analysis. As shown in Fig. 10, the respective sample groups having different contents of treatments were not sufficiently classified.

### <Example 6>

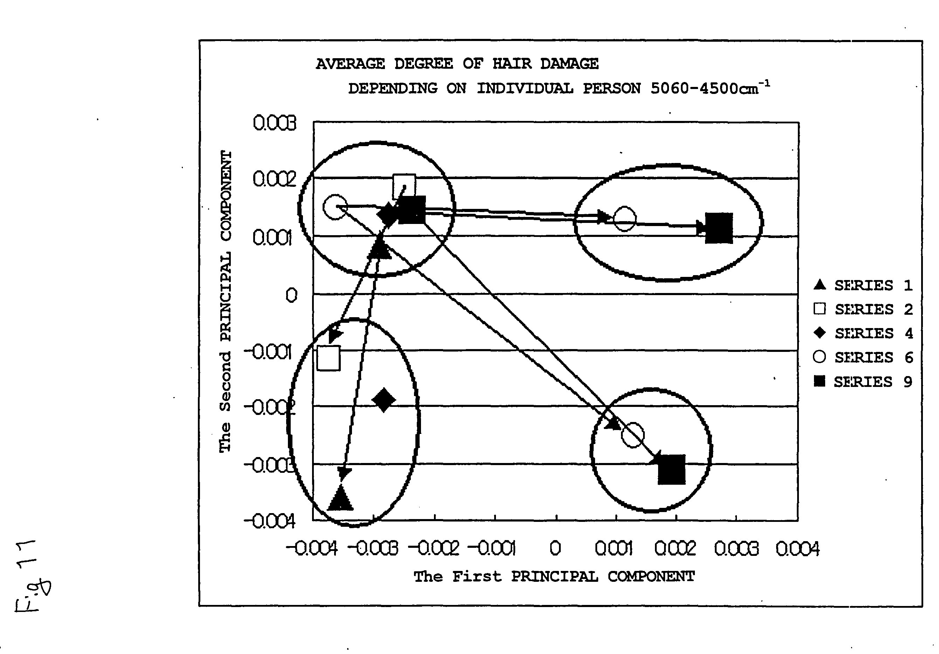

The near infrared absorption spectra of the untreated samples, and the 10% permanent treated samples (2 samples), the 3 times bleaching treated samples (3 samples) and the 10% permanent treated + bleaching treated samples (2 samples) were subjected to data processing and principal component analysis in the same manner as in Example 1. Results of the analysis were examined for how the degree of the damage of each untreated sample changed by each treatment. Fig. 11 shows the results.
As shown in Fig. 11, the relationship among untreated samples coincides with the relationship among the samples subjected to each treatment. That is, it can be understood that a hair having a lower degree of damage in an untreated state has a lower degree of damage when subjected to a permanent treatment and/or a bleaching treatment, while a hair having a higher degree of damage in an untreated state has a higher degree of damage when subj ected to a permanent treatment and/or a bleaching treatment.
Therefore, the degree of damage of hair after the treatment can be predicted from the evaluating result of the degree of damage in an untreated state. That is, the hair as to its degree of likelihood to be easily damaged by the treatment can be determined.

### INDUSTRIAL APPLICABILITY

According to the method of the present invention, the state of degree of a hair damage can be monitored. In addition, through evaluating the state of a hair damage by the method of the present invention, a cosmetic or treatment method suitable for the hair can be selected, or an effect of a certain cosmetic or treatment to be applied on the hair can be predicted.

## Claims

1. A method of evaluating a degree of at least one of a damage of a hair caused by a permanent treatment and a damage of the hair caused by an oxidation treatment, based on a near infrared absorption spectrum of the hair, comprising the steps of:
obtaining a correlation between the degree of at least one of the damage of a hair caused by the permanent treatment and the damage of the hair caused by the oxidation treatment, and a result of multivariate analysis of near infrared absorption spectrum (wavenumber region: 8,000 to 4,500 cm⁻¹) of the hair, based on results of the multivariate analysis of the near infrared absorption spectra of two or more kinds of hairs, a degree of at least one of damages caused by a permanent treatment and by an oxidation treatment is known;
obtaining a near infrared absorption spectrum of a hair which is an evaluating object, a degree of at least one of damages caused by the permanent treatment and by the oxidation treatment is unknown; and
evaluating the degree of at least one of the damage caused by the permanent treatment and the damage caused by the oxidation treatment of the hair of the evaluating object, based on the near infrared absorption spectrum obtained in the step 2) and based on the obtained correlation.

2. The method according to claim 1, wherein the multivariate analysis is an analysis which uses a principal component analysis (PCA), SIMCA, or KNN.

3. The method according to claim 1 or 2, wherein the damage caused by the oxidation treatment is a damage caused by a bleaching treatment.

4. The method according to claim 1, wherein the method is for determining a degree of the permanent treatment and/or an oxidation treatment applied to the hair.

5. The method according to claim 1, wherein the method is for determining the hair as to its degree of likelihood to be easily damaged by the permanent treatment and/or the oxidation treatment.
